# EUROPEAN PATENT SPECIFICATION

(11) **EP 1 231 985 B1**
(45) Date of publication and mention of the grant of the patent: **14.01.2004**
(21) Application number: 99965613.5
(22) Date of filing: 15.11.1999
(51) Int. Cl.: A62B 9/00

(54) **MAINTENANCE AND ANALYSIS MODULE FOR A GAS SYSTEM**
MODUL FÜR DIE WARTUNG UND AUSWERTUNG VON EINEM GASSYSTEM
MODULE DE MAINTENANCE ET D'ANALYSE POUR SYSTEME A GAZ

(43) Date of publication of application: 21.08.2002
(73) Proprietor: AMON AB, 360 13 Urshult (SE)
(72) Inventor: LODGE, Denis, S-360 13 Urshult (SE)
(74) Representative: Andersson, Per-Olof
(86) International application number: PCT/SE1999/002081
(87) International publication number: WO 2001/036049

(56) References cited:
- US-A- 5 694 922
- US-A- 5 711 294

## Description

### Technical field

The invention relates to a support and analysis module for a gas system comprising at least one gas pipe through which a gas is led from a source to a user.

### Background of the invention

Within the field of health care and more specifically in hospitals where beds are available for patients who require a continuous supply of medicinal gas, such as oxygen, it is becoming increasingly common to have a gas system comprising a gas source central from which gas pipes lead to the beds. For instance, document US-A-5711294 discloses a gas system comprising all the features set out in the preamble of claim 1. Naturally this area of application places great demands both on the cleanness and reliability of the gas system. For the safety of the patients, therefore, no dangerous contamination of the gas pipes walls nor uncontrolled mixing of different gases nor sudden pressure variations may occur. This means that the gas system must be inspected regularly for contamination and if any contamination is found the system must be cleaned immediately. It is also desirable for logistics and economical reasons that occasional or continual measurements of gas consumption is carried out at department/ward level for example.

### The object of the invention

At present there is no apparatus that can be connected easily to both new and old installations and render possible the measures described above. In the light of these facts the object of the invention is to produce an apparatus which, owing to a standardised construction, is both reliable and simple to install.

### Summary of the invention

The object of the invention is realised through a support and analysis module of the above-mentioned type, which is characterised in that it has an access port to which the first section of the gas pipe coming from the source is connected; an outlet port, to which a second section of the gas pipe leading to the user is connected and a connecting unit which connects the access and outlet ports with each other, the access port comprising an entrance for the gas pipe's first section, two outlet connectors for the supply of gas from the entrance to said connecting unit and a valve mechanism at each outlet connector for regulating the gas flow to the connecting unit, the outlet port providing an exit for the second section of the gas pipe, two access connectors for the supply of gas from said connecting unit to the exit and a valve mechanism at each access connector for regulating the gas flow from the connecting unit, the connecting unit comprising first and second separate connecting pipes, each of which is connected to an outlet and an access connector for the supply of gas from the respective outlet connector to the respective access connector, said first connecting pipe functioning as a main supply and said second connecting pipe functioning as a bypass supply, which preferably is used only when the gas flow to and from the first connecting pipe has been stopped by means of the adherent valve mechanism in the access and outlet ports.

By creating an apparatus in the form of a universal module for gas systems which includes a main pipe and a bypass pipe and which by means of the valve mechanism can be individually connected and disconnected, this invention facilitates both maintenance and analysis work of the gas system, whereby analyses can be carried out without disrupting normal operations and maintenance is limited to an absolute minimum period of time.

To make the module as flexible and easy to use as possible it is preferable that said first and/or second connecting pipe/s can be detached from the access and outlet ports.

An analysis of the degree of contamination in the gas system can be done by removing a pipe coupling from the system for analysis in a laboratory and said first and/or second connecting pipe/s should contain a pipe coupling of defined length and diameter.

Filtering out contaminants from the gases in the gas system can easily be done if said first and/or second connecting pipe/s include/s a filter unit, which, by means of this invention, can be exchanged without having to turn off the system or risk a fall in pressure.

To enable continuous or occasional flow measurements said first and/or second connecting pipe/s can also include a flow gauge.

Said first and/or second connecting pipe/s can also include a pressure gauge, which makes it possible to read the pressure locally in the gas system and can, for example, set off an alarm should a sudden fall in pressure occur.

For temporary gas supply when the gas system is otherwise turned off or to enable flushing or washing of a system composed of a series of connected modules, said first and/or second connecting pipe/s can include an extra access connector for connection to another source, such as a gas cylinder or a flushing or washing fluid container or circulation pump.

For even more flexibility the access and/or outlet ports can have an extra outlet connector which connects to the gas pipe and also an extra valve mechanism for regulating the gas flow to said extra outlet connector, at which, for example, a pressure gauge can be attached to the extra outlet connector.

As very high standards are required of gas systems within the health care sector the support and analysis module according to the invention shows to its advantage when connected to a gas system in a hospital when the gas is a medicinal gas.

### Brief description of the drawing

A preferred embodiment of the invention is described in detail with reference to the enclosed schematic drawing, in which:
Fig. 1 is a perspective of the complete support and analysis module.
Fig. 2 shows a connecting pipe of the module comprising a particle filter.
Fig. 3 shows a connecting pipe of the module comprising a flow gauge.
Fig. 4 shows a connecting pipe of the module comprising an extra access connector.

### Description of the preferred embodiment

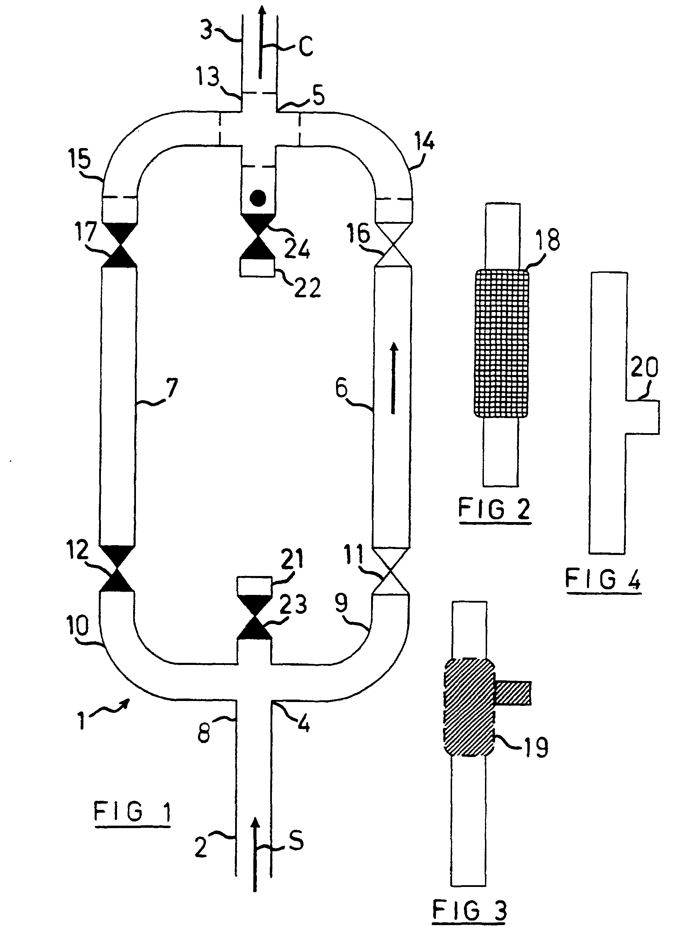

The support and analysis module 1 in Fig. 1 is primarily intended for use in hospitals. It is intended for use together with several similar modules that are mounted into a cabinet (not shown), to which the gas pipes 2, 3 for supply of different medicinal gases to the different modules are attached.

The module 1 has an access end 4, which is connected to the first gas pipe section 2, which comes from a source symbolised here by the arrow S, which also indicates the normal flow direction of the gas in the first gas pipe section 2. The module 1 also has an outlet end 5, which is connected to a second gas pipe section 3, which leads to a user, and which is symbolised here by arrow C, which also indicates the normal flow direction of the gas in the second gas pipe section 3. In addition to this the module 1 has a connecting unit 6, 7 between the access and outlet ends 4, 5 which is described below in more detail and which connects the access and outlet ends 4, 5 with each other.

The access end 4 comprises an access opening 8 for the first gas pipe section 2, two outlet connectors 9, 10 for the transport of gas from the access opening (8) to the connecting unit 6, 7 and stop valves 11, 12 at each outlet connector 9, 10 for regulating the gas flow to the connecting unit 6, 7.

The outlet end 5 comprises an outlet opening 13 for the second gas pipe section 3, two access connectors 14, 15 for transport of gas from the connecting unit 6, 7 to the outlet opening 13 and stop valves 16, 17 at each access connector 14, 15 for regulating the gas flow from the connecting unit 6, 7.

The connecting unit of the embodiment in Fig. 1 consists of first and second separate cylindrical connecting pipes 6, 7. Connecting pipes 6, 7 are, via stop valves 11, 12, 16, 17, connected to each of the outlet and access connectors 9, 10, 14, 15 for transport of gas from the respective outlet connectors 9, 10 to the respective access connectors 14, 15. The first connecting pipe 6 thus functions as a main pipe and the second connecting pipe 7 as a bypass pipe, which is preferably used only when the gas flow to and from the first connecting pipe 6 has been stopped by means of the adherent stop valves 11, 16.

The first and the second connecting pipes 6, 7 in the shown preferred embodiment of the module 1 can be detached at the stop valves 11, 12, 16, 17. The stop vales have suitable pipe couplings (not shown) for this purpose. Providing the connecting pipe 6 is used as the main pipe and its length and diameter are of suitable dimensions for the purpose of analysis, it is possible to easily detach the connecting pipe 6 and send it to a laboratory for examining the degree of contamination in the gas system.

It is also possible to fit other appropriate gas pipe sections in place of the connecting pipes 6, 7. Such a section shown in Fig. 2 can include a filter 18, such as a particle or absolute filter. Another such section shown in Fig. 3 can include a flow or pressure gauge 19. A similar section shown in Fig. 4 can include an extra access connector 20 for connection to another source, such as a reserve gas source (not shown).

It is also preferable that the access and outlet openings 4, 5 each have extra outlet connectors 21, 22 as shown in Fig. 1 which, via extra stop valves 23, 24, are connected to gas pipes 2, 3. It is possible to connect different measuring instruments to the extra outlet connectors; preferably a pressure gauge (not shown).

The person skilled in the art is aware that the module 1 described above has many various modifications within the framework of the enclosed patent requirements and that the module can be used for gas systems other than those installed in hospitals.

## Claims

1. A support and analysis module for a gas system that includes at least one pipe system (2, 3), through which a gas is led from a source (S) to a user (C), wherein said module has an access port (4), to which the first section (2) of the gas pipe coming from the source (S) is connected; an outlet port (5) to which a second section (3) of the gas pipe leading to the user is connected, and a connecting unit (6, 7) which connects the access and outlet ports (4) (5) with each other, **characterised in that** the access port (4) comprises an entrance (8) for the gas pipe's first section (2), two outlet connectors (9, 10) for the supply of gas from the entrance (8) to said connecting unit(6, 7) and valves (11, 12) at each outlet connector (9, 10) for regulating the gas flow to the connecting unit (6, 7), the outlet port (5) provides an exit (13) for the second section of the gas pipe (3), two access connectors (14, 15) for the supply of gas from said connecting unit to the exit (13) and valves(16, 17) at each access connector (14, 15) for regulating the gas flow from the connecting unit(6, 7) the connecting unit comprises first and second separate connecting pipes (6, 7) each of which is connected to an outlet and an access connector (9, 10, 14, 15) for the supply of gas from the respective outlet connectors (9, 10) to the respective access connectors (14, 15), said first connecting pipe (6) functioning as a main supply and said second connecting pipe (7) functioning as a bypass supply, which is preferably used only when the gas flow to and from the first connecting pipe (6) has been stopped by means of the adherent valves (11, 16) in the access and outlet ports (4, 5).

2. The module according to claim 1, **characterised in that** said first and/or second connecting pipe/s (6, 7) is/are connected to and detachable from the access and outlet connectors (9, 10, 14, 15).

3. The module according to claim 1 or 2, **characterised in that** said first and/or second connecting pipe/s (6, 7) comprise/s a pipe section of defined length and diameter.

4. The module according to claims 1-3, **characterised in that** said first and/or second connecting pipe/s (6, 7) comprise/s a filter unit (18).

5. The module according to claims 1-4, **characterised in that** said first and/or second connecting pipe/s (6, 7) comprise/s a flow gauge (19).

6. The module according to claims 1-5, **characterised in that** said first and/or second connecting pipe/s (6, 7) comprise/s a pressure gauge (19) .

7. The module according to claims 1-5, **characterised in that** said first and/or second connecting pipe/s (6, 7) comprise/s an extra inflow connection (20) for connection to another source.

8. The module according to claims 1-7, **characterised in that** said the access and/or outlet ports (4, 5) have extra outlet connectors (21, 22), which can be coupled to the gas pipe sections (2, 3) and extra valve mechanism (23, 24) for regulating the gas flow to said extra outlet connectors (21, 22).

9. The module according to claim 8, **characterised in that** said that a pressure gauge is connected to the extra outlet connectors (21, 22).

10. The module according to the claims 1-9, **characterised in that** the module is connected to a gas system in a hospital and that the gas is a medicinal gas.

## Patentansprüche

1. Trage- und Analysemodul für ein Gassystem, das wenigstens ein Rohrsystem (2, 3) enthält, über das ein Gas von einer Quelle (S) zu einem Nutzer (C) geleitet wird, wobei das Modul einen Zugangsanschluss (4), mit dem der erste Abschnitt (2) des Gasrohrs, der von der Quelle (S) kommt, verbunden ist; einen Auslassanschluss (5), mit dem ein zweiter Abschnitt (3) des Gasrohrs, der zu dem Nutzer führt, verbunden ist, und eine Verbindungseinheit (6, 7) aufweist, die den Zugangs- und den Auslassanschluss (4) (5) miteinander verbindet, **dadurch gekennzeichnet, dass** der Zugangsanschluss (4) einen Eintritt (8) für den ersten Abschnitt (2) des Gasrohrs, zwei Auslassverbinder (9, 10) für die Zufuhr von Gas von dem Eintritt (8) zu der Verbindungseinheit (6, 7) und Ventile (11, 12) an jedem Auslassverbinder (9, 10) zum Regulieren des Gasstroms zu der Verbindungseinheit (6, 7) umfasst, der Auslässanschtuss (5) einen Austritt (13) für den zweiten Abschnitt des Gasrohrs (3), zwei Zugangsverbinder (14, 15) für die Zufuhr von Gas von der Verbindungseinheit zu dem Austritt (13) und Ventile (16, 17) an jedem Zugangsverbinder (14, 15) zum Regulieren des Gasstroms von der Verbindungseinheit (6, 7) bereitstellt, die Verbindungseinheit ein erstes und ein zweites separates Verbindungsrohr (6, 7) umfasst, von denen jedes mit einem Auslass- und einem Zugangsverbinder (9, 10, 14, 15) für die Zufuhr von Gas von den entsprechenden Auslassverbindem (9, 10) zu den entsprechenden Zugangsverbindern (14, 15) verbunden ist, wobei das erste Verbindungsrohr (6) als eine Hauptzufuhr dient und das zweite Verbindungsrohr (7) als Umgehungszufuhr dient, die vorzugsweise nur dann verwendet wird, wenn der Gasstrom zu und von dem ersten Verbindungsrohr (6) mittels der zugehörigen Ventile (11, 16) in dem Zugangs- und dem Auslassanschluss (4, 5) unterbrochen worden ist.

2. Modul nach Anspruch 1, **dadurch gekennzeichnet, dass** das erste und/oder das zweite Verbindungsrohr (6, 7) mit den Zugangs- und den Auslassverbindem (9, 10, 14, 15) verbunden ist/sind und davon getrennt werden kann/können.

3. Modul nach Anspruch 1 oder 2, **dadurch gekennzeichnet, dass** das erste und/oder das zweite Verbindungsrohr (6, 7) einen Rohrabschnitt mit festgelegter Länge und festgelegtem Durchmesser umfasst/umfassen.

4. Modul nach den Ansprüchen 1-3, **dadurch gekennzeichnet, dass** das erste und/oder das zweite Verbindungsrohr (6, 7) eine Filtereinheit (18) umfasst/umfassen.

5. Modul nach den Ansprüchen 1-4, **dadurch gekennzeichnet, dass** das erste und/oder das zweite Verbindungsrohr (6, 7) einen Durchflussmesser (19) umfasst/umfassen.

6. Modul nach den Ansprüchen 1-5, **dadurch gekennzeichnet, dass** das erste und/oder das zweite Verbindungsrohr (6, 7) einen Druckmesser (19) umfasst/umfassen.

7. Modul nach den Ansprüchen 1-5, **dadurch gekennzeichnet, dass** das erste und/oder das zweite Verbindungsrohr (6, 7) eine Zusatz-Einströmverbindung (20) zur Verbindung mit einer anderen Quelle umfasst/umfassen.

8. Modul nach den Ansprüchen 1-7, **dadurch gekennzeichnet, dass** der Zugangsund/oder der Auslassanschluss (4, 5) Zusatz-Auslassverbinder (21, 22), die mit den Gasrohrabschnitten (2, 3) verbunden werden können, und Zusatz-Ventilmechanismen (21, 24) zum Regulieren des Gasstroms zu den Zusatz-Auslassverbindern (21, 22) aufweisen.

9. Modul nach Anspruch 8, **dadurch gekennzeichnet, dass** ein Druckmesser mit den Zusatz-Auslassverbindern (21, 22) verbunden ist.

10. Modul nach den Ansprüchen 1-9, **dadurch gekennzeichnet, dass** das Modul mit einem Gassystem in einem Krankenhaus verbunden ist und das Gas ein medizinisches Gas ist.

## Revendications

1. Module de support et d'analyse pour un système de gaz qui comprend au moins un système de conduites (2, 3) à travers lequel un gaz est conduit à partir d'une source (S) vers un utilisateur (C), dans lequel ledit module comporte un orifice d'accès (4) auquel la première section (2) de la conduite de gaz provenant de la source (S) est reliée ; un orifice de sortie (5) auquel une deuxième section (3) de la conduite de gaz conduisant à l'utilisateur est reliée, et une unité de connexion (6, 7) qui relie les orifices d'accès et de sortie (4, 5) entre eux, **caractérisé en ce que** l'orifice d'accès (4) comprend une entrée (8) pour la première section (2) de la conduite de gaz, deux connecteurs de sortie (9, 10) pour l'alimentation en gaz à partir de l'entrée (8) vers ladite unité de connexion (6, 7) et des vannes (11, 12) à chaque connecteur de sortie (9, 10) pour réguler l'écoulement de gaz vers l'unité de connexion (6, 7), l'orifice de sortie (5) fournit une sortie (13) pour la deuxième section de la conduite de gaz (3), deux connecteurs d'accès (14, 15) pour l'alimentation en gaz à partir de ladite unité de connexion vers la sortie (13) et les vannes (16, 17) à chaque connecteur d'accès (14, 15) pour réguler l'écoulement de gaz provenant de l'unité de connexion (6, 7), l'unité de connexion comprend une première et une deuxième conduites de connexion séparées (6, 7) dont chacune est reliée à une sortie et à un connecteur d'accès (9, 10, 14, 15) pour l'alimentation en gaz à partir des connecteurs de sortie (9, 10) respectifs vers les connecteurs d'accès (14, 15) respectifs, ladite première conduite de connexion (6) fonctionnant comme une alimentation principale et ladite deuxième conduite de connexion (7) fonctionnant comme une alimentation de dérivation, qui est de préférence utilisée seulement lorsque l'écoulement de gaz vers et à partie de la première conduite de connexion (6) a été arrêté à l'aide des vannes (11, 16) adhérentes dans les orifices d'accès et de sortie (4, 5).

2. Module selon la revendication 1, **caractérisé en ce que** ladite première et/ou deuxième conduite(s) de connexion (6, 7) est/sont connectée(s) et peut/peuvent être détachée(s) des connecteurs d'accès et de sortie (9, 10, 14, 15).

3. Module selon la revendication 1 ou 2, **caractérisé en ce que** ladite première et/ou deuxième conduite(s) de connexion (6, 7) comprend/comprennent une section de conduite de longueur et de diamètre défini.

4. Module selon les revendications 1 à 3, **caractérisé en ce que** ladite première et/ou deuxième conduite(s) de connexion (6, 7) comprend/comprennent une unité de filtre (18).

5. Module selon les revendications 1 à 4, **caractérisé en ce que** ladite première et/ou deuxième conduite(s) de connexion (6, 7) comprend/comprennent une jauge de débit (19).

6. Module selon les revendications 1 à 5, **caractérisé en ce que** ladite première et/ou deuxième conduite(s) de connexion (6, 7) comprend/comprennent une jauge de pression (19).

7. Module selon les revendications 1 à 5, **caractérisé en ce que** ladite première et/ou deuxième conduite(s) de connexion (6, 7) comprend/comprennent une connexion (20) supplémentaire d'amenée pour une connexion à une autre source.

8. Module selon les revendications 1 à 7, **caractérisé en ce que** lesdits orifices d'accès et/ou de sortie (4, 5) ont des connecteurs de sortie (21, 22) supplémentaires, qui peuvent être raccordés aux sections de la conduite de gaz (2, 3) et un mécanisme de vanne (23, 24) supplémentaire pour réguler l'écoulement de gaz vers lesdits connecteurs de sortie (21, 22) supplémentaires.

9. Module selon la revendication 8, **caractérisé en ce que** ladite jauge de pression est reliée aux connecteurs de sortie (21, 22) supplémentaires.

10. Module selon les revendications 1 à 9, **caractérisé en ce que** le module est relié à un système de gaz dans un hôpital et **en ce que** le gaz est un gaz médicinal.
